# EUROPEAN PATENT SPECIFICATION

(11) **EP 3 379 248 B1**
(45) Date of publication and mention of the grant of the patent: **23.09.2020**
(21) Application number: 16866421.7
(22) Date of filing: 18.11.2016
(51) Int. Cl.: C12Q 1/34, G01N 33/50

(54) **ARGINASE ACTIVITY MEASUREMENT METHOD, ARGINASE ACTIVITY DETECTION KIT, ARGINASE-RELATED DISEASE DETECTION KIT, AND ARGINASE INHIBITOR OR ACTIVE AGENT SCREENING METHOD**
VERFAHREN ZUR MESSUNG DER ARGINASE-AKTIVITÄT, KIT FÜR DEN NACHWEIS DER ARGINASE-AKTIVITÄT, KIT FÜR DEN NACHWEIS VON ARGINASE-ASSOZIIERTEN ERKRANKUNGEN UND SCREENING-VERFAHREN FÜR ARGINASE-INHIBITOR ODER WIRKSTOFF
PROCÉDÉ DE MESURE DE L'ACTIVITÉ DE L'ARGINASE, KIT DE DÉTECTION DE L'ACTIVITÉ DE L'ARGINASE, KIT DE DÉTECTION DE MALADIES ASSOCIÉES À L'ARGINASE, ET PROCÉDÉ DE CRIBLAGE À LA RECHERCHE D'AGENTS ACTIFS OU D'INHIBITEURS DE L'ARGINASE

(30) Priority: 18.11.2015 JP 2015225939; 29.01.2016 JP 2016015903; 31.03.2016 WO PCT/JP2016/061637; 03.10.2016 JP 2016195933
(43) Date of publication of application: 26.09.2018
(73) Proprietor: Tokyo Ohka Kogyo Co., Ltd., Kanagawa 211-0012 (JP); Tabata, Yasuhiko, Uji-shi Kyoto 611-0024 (JP)
(72) Inventor: MAENO, Emi, Kawasaki-shi Kanagawa 211-0012 (JP)
(74) Representative: Plasseraud IP
(86) International application number: PCT/JP2016/084208
(87) International publication number: WO 2017/086421

(56) References cited:
- WO-A2-2015/003149
- JP-A- 2010 500 973
- JP-A- 2015 516 397
- US-A1- 2008 021 097
- US-A1- 2009 031 457
- US-A1- 2015 080 341
- ALMY, J ET AL.: 'Urea Analysis for Wines' J. AGRIC. FOOD CHEM. vol. 37, 1989, pages 968 - 970, XP055488045

## Description

### Technical Field

The present invention relates to an arginase activity measurement method, an arginase activity detection kit, an arginase-related disease detection kit, and an arginase inhibitor or active agent screening method.

### Background Art

Arginase is an enzyme catalyzing the final step in the urea cycle, and arginase converts L-arginine to L-ornithine and urea and plays an important role in the disposal of ammonium ions from the body. Furthermore, arginase has two isoforms, namely, type I and type II isoforms. Type I isoform exists mainly in the liver and has an important function in the urea cycle. Type II isoform exists in organs including the kidneys and controls the concentrations of arginine and ornithine.

It has been reported that deficiency of arginase causes serious symptoms such as neurological disorders, dementia, and hyperammonemia, and measurement of the arginase activity is essential for the research of the urea metabolic system. Furthermore, it has been reported that arginase has effects of suppressing macrophage anti-inflammatory action and apoptosis (cell death), and in recent years, arginase is increasingly utilized as an index for the effects.

Regarding the arginase activity measurement method, methods involving the measurement of (1) the amount of production of ornithine, (2) the amount of production of urea, (3) the amount of production of ammonia, based on a series of enzyme reactions, in which arginase degrades arginine into ornithine and urea, and urea is further decomposed into ammonia and carbon dioxide, may be mentioned (see, for example, Patent Document 1).

### Citation List

### Patent Literature

[Patent Document 1] Japanese Unexamined Patent Application, First Publication No. S63-59899
[Patent Document 21 The document US 2015/080341 A1 refers to ring constrained analogs as arginase inhibitors.
[Patent Document 31 The document US 2008/021097 A1 relates to composition and methods of use thereof, for treating conditions associated with elevated arginase levels and/or activity.
[Patent Document 41 The document WO 2015/003149 A2 relates to soluble CD33 for treating myelodysplastic syndromes.
[Patent Document 5] The document US 2009/031457 A1 relates to genes, proteins and methods comprising molecules that alter amino acid levels.

### Summary of Invention

### Technical Problem

Patent Document 1 discloses, in regard to the (1) method for measuring the amount of production of ornithine, a method of detecting ornithine by high performance liquid chromatography (HPLC). Thus, a measuring apparatus for HPLC is necessary, and the measurement time takes longer. Furthermore, in regard to the (2) method for measuring the amount of production of urea, since the commercially available reagents for urea detection are sparingly water-soluble and the operation is even more complicated, measurement is limited to several dozens of samples, and large amounts of samples and large amounts of reagents are required. Furthermore, in the (3) method for measuring the amount of production of ammonia, since an enzyme reaction involving a urease is needed, the operation becomes complicated.

The present invention was achieved in view of the circumstances described above, and the invention provides a highly sensitive, simple and convenient arginase activity measurement method.

### Solution to Problem

Disclosed is an arginase activity measurement method, including: a step of adding a sample including arginase and a solution including a divalent cation to a reaction vessel, and thereby activating the arginase; a step of adding an arginine sol uti on to the sample including the activated arginase, and performing an enzyme-substrate reaction; and a step of adding an acidic solution including a urea detection reagent and havi ng a pH of from 1.0 to 4.0 to the sample subjected to the enzyme-substrate reaction, and simultaneously performing deactivation of enzyme and a urea detection reaction.

A first embodiment of the present invention is an arginase activity measurement method, including: a step of adding a sample including arginase and a solution including a divalent cation to a reaction vessel, and thereby activating arginase; a step of adding an arginine solution to the sample including the activated arginase, and performing an enzyme-substrate reaction; and a step of adding an acidic solution including a-isonitrosopropiophenone as a urea detection reagent and physiological saline or distilled water to the sample subjected to the enzyme-substrate reaction, and simultaneously performing deactivation of enzyme and a urea detection reaction.

A second embodiment of the present invention is an arginase activity detection kit including an acidic solution including a-isonitrosopropiophenone as a urea detection reagent and physiological saline or distilled water.

Also disclosed is an arginase activity detection kit including a solution including arginine and a divalent cation; and an acidic solution including a urea detection reagent and having a pH of from 1.0 to 4.0.

Also disclosed is an arginase-related disease detection kit including a solution including arginine and a divalent cation; and an acidic solution including a urea detection reagent and havi ng a pH of from 1.0 to 4.0.

A third embodiment of the present invention is an arginase inhibitor or active agent screening method, including: a step of adding a sample including arginase and a solution including a divalent cation to a reaction vessel and thereby activating arginase, in the presence or absence of a test substance; a step of addi ng an argi ni ne sol uti on to the sample including the activated arginase, and performing an enzyme-substrate reaction; a step of adding a -isonitrosopropiophenone as a urea detection reagent and physiological saline or distilled water to the sample subjected to the enzyme-substrate reaction, and simultaneously performing deactivation of enzyme and a urea detection reaction; and a step of determining that in a case in which the concentration of urea detected in the presence of a test substance is low compared to the concentration of urea detected in the absence of the test substance, the test substance is an inhibitor of arginase, and that in a case in which the concentration of urea detected in the presence of the test substance is high compared to the concentrati on of urea detected in the absence of the test substance, the test substance is an active agent of arginase.

### Advantageous Effects of Invention

According to the present invention, arginase activity can be measured simply and conveniently with high sensitivity.

### Brief Description of Drawings

FIG. 1 is a diagram showing an outline configuration of the arginase activity measurement method accordi ng to an embodi ment.
FIG. 2 is a diagram showing the measurement results for arginase activity in Example 1.
FIG. 3 is a diagram showing the measurement results for arginase activity of Comparative Example 1.
FIG. 4A is an image showi ng the measurement results for arginase activity in Example 2.
FIG. 4B is an image showi ng the measurement results for arginase activity in Comparative Example 2.
FIG. 5A is a diagram showing the measurement results for arginase activity obtained in Example 3, when the urea detection reaction was accelerated by heating the reaction system for 1 hour and 45 mi nutes in the urea detection reaction step.
FIG. 5B is a diagram showi ng the measurement results for the arginase activity obtained in Example 3, when the urea detection reaction was accelerated by heating the reaction system for 2 hours and 15 minutes in the urea detection reaction step.
FIG. 6A is a diagram showing the measurement results for arginase activity obtained in Comparative Example 3, when the urea detection reagent and the acidic solution were mixed immediately before the step for enzyme deactivation and urea detection reaction, and the mixture was left to stand for 10 mi nutes at room temperature in the step for enzyme deactivation and urea detection reaction so as to develop color.
FIG. 6B is a diagram showi ng the measurement results for the arginase activity obtained in Comparative Example 3, when the urea detection reagent and the acidic solution were mixed immediately before the step for enzyme deactivation and urea detection reaction, and the mixture was left to stand for 120 mi nutes at room temperature in the step for enzyme deactivation and urea detection reaction so as to develop color.
FIG. 7A is a diagram showing the measurement results for arginase activity obtained in Example 4, when an acidic solution including a urea detection reagent that had been kept for 24 hours after production was used, and the urea detection reaction was accelerated by heati ng the reaction system for 1 hour and 45 mi nutes in the urea detection reacti on step.
FIG. 7B is a diagram showi ng the measurement results for the arginase activity obtained in Example 4, when an acidic solution including a urea detection reagent that had been kept for 24 hours after production was used, and the urea detection reaction was accelerated by heati ng the reaction system for 2 hours and 15 mi nutes in the urea detection reaction step.

### D escri pti on of Embodiments

### <Arginase activity measurement method (1)>

According to an embodiment, the present description provides an arginase activity measurement method, including a step of adding a sample including arginase and a solution including a divalent cation to a reaction vessel, and thereby activating arginase; a step of addi ng an argi ni ne solution to the sample of the activated arginase, and performing an enzyme-substrate reaction; and a step of adding an acidic solution including a urea detection reagent and having a pH of from 1.0 to 4.0 to the sample subjected to the enzyme-substrate reaction, and si multaneously performing deactivation of enzyme and a urea detection reaction.

A ccordi ng to the measurement method of the present embodi ment, the arginase activity can be measured simply and conveniently with high sensitivity.

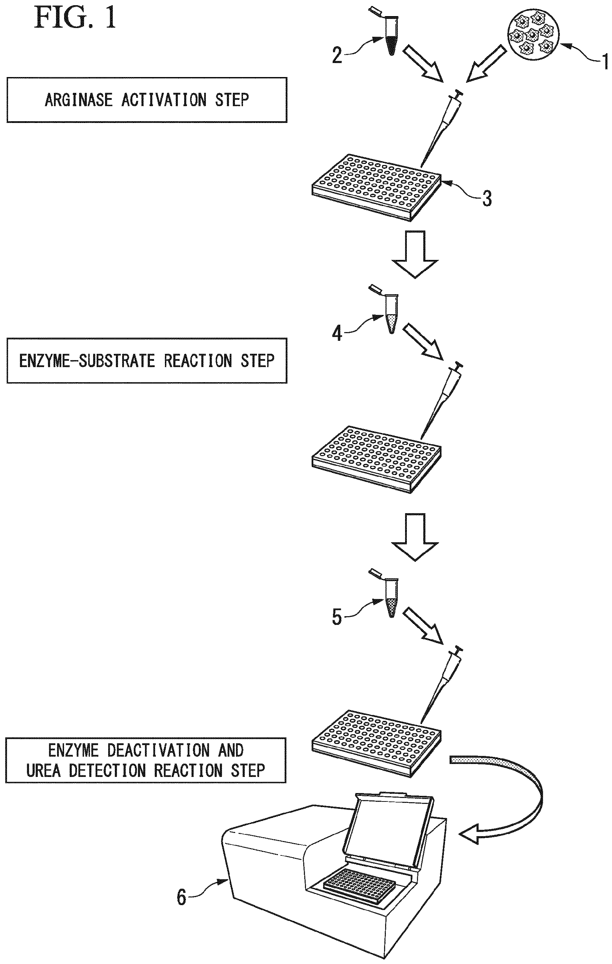

The measurement method of the present embodi ment will be explained with reference to the drawings. FIG. 1 is a diagram showing an outline configuration of the arginase activity measurement method according to an embodiment.

### [Arginase activation step]

First, a sample including arginase is produced, and the sample is added to a reaction vessel together with a solution including a divalent cation. Subsequently, in order to further enhance the activity of arginase, the reaction system may be incubated for about 10 mi nutes at a temperature at which enzyme is not deactivated (for example, a temperature of 55°C or higher and lower than 70°C).

The sample including arginase is not particularly limited; however, examples of the sample include body fluids such as blood, saliva, tear, and sweat; biological samples such as urine; a suspension of animal cells (for example, liver cells), and a disrupted cell suspension of animal cells. The sample including arginase may also be a dilution that has been diluted with a buffer solution such as physiological saline, or with distilled water or the like.

Examples of the divalent cation include Ca²⁺, Mg²⁺, Zn²⁺, and Mn²⁺. Among them, the divalent cation is preferably Mn²⁺. The solution including a divalent cation may be any solution in which a salt, a hydrate or the like containing the divalent cation described above is dissolved, and there are no particular limitations. Furthermore, the solution may also be a dilution that has been diluted with a buffer solution such as physiological saline, or with distilled water or the like. The enzymatic activity of arginase can be enhanced by adding a solution including a divalent cation to the sample including arginase.

The concentration of the divalent cation in the solution may be, for example, from 1 mM to 100 mM, may be from 3 mM to 50 mM, and may be from 5 mM to 30 mM.

The reaction vessel is not particularly limited as long as the arginase activity can be measured with the vessel, and examples include reaction vessels made of glass, metals, and plastics. The shape of the reaction vessel is not particularly limited, and examples include a slide glass shape, a microplate shape, and a disc shape.

A mong them, a microplate shape is preferred because a large number of samples can be measured. An example of the microplate may be a microplate having an arbitrary number of wells disposed therein. The number of wells may be, for example, 24, 96, 384, or 1,536, per sheet of the plate. The reaction vessel may constitute a micro-flow channel device equipped with fine flow channels. The size of the reaction vessel does not matter as long as the size is in the range that is applicable to the apparatus used.

### [E nzyme-substrate reaction step]

Next, an arginine solution is added as a substrate to the sample including activated arginase, and an enzyme-substrate reaction is carried out. The reaction temperature is preferably from 20° C to 45° C, more preferably from 30° C to 40° C, and most preferably from 37° C to 40° C. The reaction time is preferably from 30 mi nutes to 3 hours, and particularly preferably 2 hours. The pH at the ti me of the reaction is preferably from pH 8.5 to 10.5, and particularly preferably from pH 9.0 to 10.0.

The arginine solution may be any solution including arginine (preferably, L-arginine), and there are no particular limitations. The arginine solution can be produced by dissolving a salt or a hydrate containing arginine in a solvent such as distilled water. Furthermore, the arginine solution may be a dilution that has been diluted with a buffer solution such as physiological saline, or with distilled water or the like.

### [Enzyme deactivation and urea detection reaction step]

Next, an acidic solution including a urea detection reagent and having a pH of from 1.0 to 4.0 is added to the sample subjected to the enzyme-substrate reaction, and deactivation of enzyme and a urea detection reaction are simultaneously carried out.

The urea detection reagent is not particularly limited as long as the reagent is capable of detecting urea. Examples include a-diketone, p-dimethylaminobenzaldehyde, and xanthydrol. Among them, a-diketone is preferred. Examples of the a-diketone include a-isonitrosopropiophenone and diacetyl monooxime. Among them, a-isonitrosopropiophenone is preferred.

The acidic solution having a pH of from 1.0 to 4.0 is not particularly limited as long as the pH can be adjusted to the range described above. Examples of the acid that can be used include hydrochloric acid, phosphoric acid, sulfuric acid, nitric acid, and salts and hydrates thereof. A mixture of two or more kinds of the above-descri bed acids may also be used. Furthermore, the acidic solution may also be a dilution that has been diluted with a buffer solution such as physiological saline, or with distilled water or the like.

When the urea detection reagent and the acidic solution having a pH of from 1.0 to 4.0 are mixed in advance, the deactivation of enzyme and the detection of urea can be carried out by a single operation, and therefore, the operation can be made more efficient. Furthermore, since the amount of addition of the solution can be reduced, the variation between the amount of addition of the solution and the amount of addition of the urea detection reagent can be reduced. When an acidic solution including the urea detection reagent and having a pH in the range described above is combined with heating in the subsequent urea detection reaction accelerating step, enzyme reaction and deactivation of enzyme can be sufficiently carried out.

The acidic solution including a urea detection reagent and having a pH of from 1.0 to 4.0 can be produced by, for example, the following method. First, a urea detection reagent and an acid, or a salt or a hydrate of the acid, are mixed, together with a buffer solution such as physi ological saline, or with distilled water or the like, as necessary. Furthermore, in a case in which it is relatively difficult for the urea detection reagent to be dissolved in the acidic solution, it is preferable to heat the mixture at the time of mixing. By heating, the urea detection reagent can be completely dissolved, and an acidic solution including the urea detection reagent and having a pH of from 1.0 to 4.0 can be produced.

The concentration of the urea detection reagent in the solution is preferably from 0.05% by weight to 1.00% by weight, more preferably from 0.08% by weight to 0.70% by weight, and even more preferably from 0.10% by weight to 0.66% by weight.

### [Urea detecti on reaction accelerating step]

After the addition of the acidic solution including a urea detection reagent and having a pH of from 1.0 to 4.0, the mixture may be incubated while being heated, and thereby the urea detecti on reaction may be accelerated. The heati ng temperature is preferably from room temperature to 100°C, and particularly preferably from 90°C to 100°C. The heating time is 30 minutes or longer, and is particularly preferably from 30 mi nutes to 12 hours. The heati ng temperature and the heati ng time can be regulated as appropriate according to the content of arginase. By i ncubati ng the reaction system while heati ng the system, the reaction caused by the urea detection reagent can be accelerated, and detection can be performed more rapidly.

Detection of urea can be carried out using a colorimetric method of utilizing a reaction between urea and the urea detection reagent. When the reaction proceeds and color is sufficiently developed, the reaction system is mounted in the measuring apparatus, and the absorbance is measured. The measuring apparatus is not particularly limited as long as the measuring apparatus can measure absorbance. Examples include an absorptiometer (spectrophotometer) and a microplate reader.

### [Measurement value calibration step]

Furthermore, the measurement value of the arginase activity may also be calibrated by measuring the protei n concentrati on in the sample.

The method for measuring the protein concentration is not particularly limited, and examples include an ultraviolet absorption method, Bradford's method (Coomassie Blue method), Lowry's method (phenol reagent method), and a bicinchoninic acid method (BCA method).

When the protein concentration in the sample is measured, and the measurement value is cal i brated, a more accurate measurement value of arginase activity can be obtained.

### <Arginase activity measurement method (2)>

According to an embodiment, the present invention provides an arginase activity measurement method, including a step of adding a sample including arginase and a solution including a divalent cation to a reaction vessel, and thereby activating arginase; a step of adding an arginine solution to the sample including activated arginase, and performing an enzyme-substrate reaction; and a step of adding an acidic solution including a-isonitrosopropiophenone as a urea detection reagent and physiological saline or distilled water to the sample subjected to the enzyme-substrate reaction, and simultaneously performing deactivation of enzyme and a urea detection reaction.

A ccordi ng to the measurement method of the present embodi ment, the arginase activity can be measured simply and conveniently with high sensitivity.

In regard to the measurement method of the present embodiment, the arginase activation step and the enzyme-substrate reaction step may be carried out si mi larly to the <Arginase activity measurement method (1)> described above.

### [Enzyme deactivation and urea detection reaction step]

Next, an acidic solution including a-isonitrosopropiophenone as a urea detection reagent is added to the sample subjected to the enzyme-substrate reaction, and deactivation of enzyme and a urea detection reaction are simultaneously carried out.

The pH of the acidic solution may be 4.0 or lower, and a solution having strong acidity, for which it is difficult to measure the pH using a pH meter having a glass electrode, may also be used. Examples of a usable acid include hydrochloric acid, phosphoric acid, sulfuric acid, nitric acid, and salts or hydrates thereof. A mixture of two or more kinds of the above-mentioned acids may also be used. Furthermore, the acidic solution is a dilution that has been diluted with a buffer solution such as physiological saline, or with distilled water or the like.

When a-isonitrosopropiophenone and the acidic solution are mixed in advance, the deactivation of enzyme and the detection of urea can be carried out by a one-time operation, and therefore, the operation can be made more efficient.

Furthermore, the amount of addition of the solution can be reduced, and the variation between the amount of addition of the solution and the amount of addition of the urea detection reagent can be reduced. Furthermore, when the acidic solution including a-isonitrosopropiophenone as a urea detection reagent is combined with heating in the subsequent urea detection reaction accelerating step, the enzyme reaction and deactivation of enzyme can be sufficiently carried out.

The acidic solution including a-isonitrosopropiophenone as a urea detection reagent can be produced by, for example, the following method. First, a -isonitrosopropiophenone and an acid, or a salt or a hydrate of the acid, are mixed, together with a buffer solution such as physiological saline, or with distilled water or the like, as necessary. In a case in which it is relatively difficult for a-isonitrosopropiophenone to be dissolved in the acidic solution, it is preferable to heat the mixture at the time of mixing. By heati ng, a -isonitrosopropiophenone can be completely dissolved, and the acidic solution including a-isonitrosopropiophenone as a urea detection reagent can be produced.

T he concentration of the urea detection reagent in the solution is preferably from 0.05% by weight to 1.00% by weight, more preferably from 0.08% by weight to 0.70% by weight, and even more preferably from 0.10% by weight to 0.66% by weight.

Subsequently, in regard to the urea detection reaction accelerating step, the process may be carried out in the same manner as in the <Arginase activity measurement method (1)>. Furthermore, the measurement method of the present embodi ment may include a measurement value calibration step, and the step may be carried out in the same manner as in the <A rgi nase activity measurement method (1)> descri bed above.

T he measurement method of the present embodi ment is capable of making efficient measurement by using small amounts of samples and reagents, as disclosed above. There has been hitherto a problem that the urea detection reagent is discolored over ti me and the background value becomes high; however, in the measurement method of the present embodiment, an acidic solution including a urea detection reagent and having a pH in the range described above can be produced in advance and stored, and thus, measurement can be made with higher sensitivity in a state in which the background value is low. T herefore, while measurement is limited to several dozens of samples in a single time of analysis in the conventional methods, a large number of samples such as several hundred samples or more can be measured in the measurement method of the present embodiment.

### <Arginase activity detection kit (1)>

According to an embodiment, the present invention provides an arginase activity detection kit including an acidic solution including a-isonitrosopropiophenone as a urea detection reagent and physiological saline or distilled water.

When the detection kit of the present embodiment is used, the arginase activity can be detected simply and conveniently with high sensitivity.

In regard to the detection kit of the present embodiment, the acidic solution includes a-isonitrosopropiophenone as a urea detection reagent.

In regard to the detection kit of the present embodiment, the pH of the acidic solution may be4.0 or lower, and a solution having strong acidity, for which it is difficult to measure the pH using a pH meter having a glass electrode, may also be used. Examples of a usable acid include hydrochloric acid, phosphoric acid, sulfuric acid, nitric acid, and salts or hydrates thereof. A mixture of two or more kinds of the above-mentioned acids may also be used. Furthermore, the acidic solution is a dilution that has been diluted with a buffer solution such as physiological saline, or with distilled water or the like.

Since the acidic solution including a-isonitrosopropiophenone as a urea detection reagent is in a state of having been produced in advance, the deactivation of enzyme and the detection of urea can be carried out by a single ti me of operation, and therefore, the operation can be made more efficient. Also, the amount of addition of the solution can be reduced, and the variation between the amount of addition of the solution and the amount of additi on of the urea detecti on reagent can be reduced. When the acidic solution including a-isonitrosopropiophenone as a urea detection reagent is combined with heating for accelerating the urea detection reaction, the enzyme reaction and the deactivation of enzyme can be sufficiently carried out.

### <Arginase activity detection kit (2)>

Also disclosed is an arginase activity detection kit including a solution including argi nine and a divalent cation, and an acidic solution including a urea detection reagent and havi ng a pH of from 1.0 to 4.0.

When the detection kit of the present embodiment is used, the arginase activity can be detected simply and conveniently with high sensitivity.

In regard to the detection kit of the present embodi ment, argi ni ne is not particularly limited in connection with the origin or the like as long as the arginine can be degraded by arginase as a substrate; however, the arginine is preferably L-arginine. Furthermore, the argi ni ne may also be in the form of a salt or a hydrate. The argi ni ne may also be in a powder form, or may be in the form of a solution that has been diluted with an appropriate solvent (for example, a buffer solution such as physiological saline, or distilled water) or the like.

In regard to the detection kit of the present embodi ment, examples of the divalent cation include cations similar to those mentioned above, and above all, the divalent cation is preferably Mn²⁺. The solution including a divalent cation may be any solution in which a salt, a hydrate or the like containing the above-mentioned divalent cation is dissolved, and there are no particular limitations. Furthermore, the solution may be a dilution that has been diluted with a buffer solution such as physiological saline, or with distilled water or the like. The enzymatic activity of arginase can be increased by adding a solution including a divalent cation to the sample including arginase.

In regard to the detection kit of the present embodi ment, the urea detection reagent is not particularly limited as long as the reagent is capable of detecting urea, and examples include reagents similarto those listed as examples in the section <Arginase activity measurement method (1)>. Among them, a-isonitrosopropiophenone is preferred.

In regard to the detection kit of the present embodiment, the acidic solution having a pH of from 1.0 to 4.0 is not particularly limited as long as the acidic solution is capable of adjusting the pH to the range described above. Examples of a usable acid include hydrochloric acid, phosphoric acid, sulfuric acid, nitric acid, and salts or hydrates thereof. A mixture of two or more kinds of the above-mentioned acids may also be used. Furthermore, the solution may also be a dilution that has been diluted with a buffer solution such as physiological saline, or with distilled water or the like.

Since the acidic solution including a urea detection reagent and having a pH of from 1.0 to 4.0 is in a state of having been produced in advance, the deactivation of enzyme and the detection of urea can be carried out by a single time of operation, the operation can be made more efficient. Furthermore, the amount of addition of the solution can be reduced, and the variation between the amount of addition of the solution and the amount of additi on of the urea detecti on reagent can be reduced. When the acidic solution including a urea detection reagent and having a pH in the range described above is combined with heating for accelerating the urea detection reaction, the enzyme reaction and the deactivation of enzyme can be sufficiently carried out.

### <Arginase-related disease detection kit>

Also disclosed is an arginase-related disease detection kit including a solution including argi nine and a divalent cation, and an acidic solution including a urea detecti on reagent and having a pH of from 1.0 to 4.0.

When the argi nase-rel ated disease detection kit of the present embodiment is used, an argi nase-rel ated disease can be detected simply and conveniently with high sensitivity.

According to the present specification, examples of the "argi nase-rel ated disease" include cardiac diseases, systemic hypertension, pulmonary hypertension, ischemia and reperfusion damage, peripheral vascular diseases, peripheral arterial diseases, subarachnoid hemorrhage, erectile dysfunction, autoimmune encephalomyelitis, chronic renal failure, gastrointestinal motility disorder, gastric cancer, reduced hepatic blood flow, insufficient hepatic blood flow, cerebral vasospasm, and combinations thereof.

In regard to the arginase-related disease detection kit of the present embodiment, arginine is not particularly limited in connection with the origin or the like as long as the arginine can be degraded by arginase as a substrate; however, the arginine is preferably L-arginine. Furthermore, the arginine may also be in the form of a salt or a hydrate. The arginine may also be in a powder form, or may be in the form of a solution that has been diluted with an appropriate solvent (for example, a buffer solution such as physiological saline, or distilled water) or the like.

In regard to the arginase-related disease detection kit of the present embodiment, examples of the divalent cation include cations similar to those mentioned above, and above all, the divalent cation is preferably Mn²⁺. The solution including a divalent cation may be any solution in which a salt, a hydrate or the like containing the divalent cation described above is dissolved, and there are no particular limitations. Furthermore, the solution may also be a dilution that has been diluted with a buffer solution such as physiological saline, or with distilled water or the like. The enzymatic activity of arginase can be enhanced by adding a solution including a divalent cation to the sample including arginase.

In regard to the arginase-related disease detection kit of the present embodiment, the urea detection reagent is not particularly limited as long as the reagent is capable of detecting urea, and examples include the reagents similar to those listed as examples in the section <Arginase activity measurement method (1)>. Among them, a-isonitrosopropiophenone is preferred.

In regard to the arginase-related disease detection kit of the present embodiment, the acidic solution having a pH of from 1.0 to 4.0 is not particularly limited as long as the acidic solution is capable of adjusting the pH to the range described above. Examples of a usable acid include hydrochloric acid, phosphoric acid, sulfuric acid, nitric acid, and salts or hydrates thereof. A mixture of two or more kinds of the above-mentioned acids may also be used. Furthermore, the acidic solution may also be a dilution that has been diluted with a buffer solution such as physiological saline, or with distilled water or the like.

Since the acidic solution including a urea detecti on reagent and having a pH of from 1.0 to 4.0 is in a state of having been produced in advance, the deactivation of enzyme and the detection of urea can be carried out by a single ti me of operation, and therefore, the operation can be made more efficient. Also, the amount of addition of the solution can be reduced, and the variation between the amount of addition of the solution and the amount of additi on of the urea detection reagent can be reduced. By adjusting the pH to the range described above, the enzyme reaction and the deactivation of enzyme can be sufficiently carried out.

### <an arginase inhibitor or active agent screening method>

According to an embodiment, the present invention provides a screening method including: a step of adding a sample including arginase and a solution including a divalent cati on to a reacti on vessel and thereby activating arginase, in the presence or the absence of a test substance; a step of adding an arginine solution to the sample including activated arginase, and performing an enzyme-substrate reaction; a step of adding an acidic solution including a-isonitrosopropiophenone as a urea detection reagent and physiological saline or distilled water to the sample subjected to the enzyme-substrate reaction, and simultaneously performing deactivation of enzyme and a urea detection reaction; and a step of determining that in a case in which the concentration of urea detected in the presence of a test substance is low compared to the concentrati on of urea detected in the absence of the test substance, the test substance is an inhibitor of arginase, and that in a case in which the concentrati on of urea detected in the presence of the test substance is high compared to the concentration of urea detected in the absence of the test substance, the test substance is an active agent of arginase.

A ccordi ng to the screeni ng method of the present embodi ment, an arginase i nhi bitor or an arginase active agent can be screened simply and conveniently.

### [Arginase activation step]

First, a sample including arginase is produced in the presence or absence of a test substance, and the sample is added to a reaction vessel, together with a solution including a divalent cation. Subsequently, in order to increase the arginase activity, the mixture may be incubated for about 10 mi nutes at a temperature at which enzyme is not deactivated (for example, a temperature of 55° C or higher and lower than 70° C).

In regard to the screeni ng method of the present embodi ment, the "test compound" is not particularly limited, and examples include expression products of a gene library, a synthetic low molecular weight compound library, a pepti de library, an antibody, a bacterial released substance, an extract and a culture supernatant of a cell (a microorganism, a plant cell, or an animal cell), a purified or partially purified polypeptide, an extract of a marine organism, a plant or an animal, soil, and a random bacteriophage peptide display library.

The sample including arginase is not particularly limited, and examples include samples similar to those listed as examples in the section <Arginase activity measurement method (1)>.

Examples of the divalent cation include cations similar to those listed as examples in the section <Arginase activity measurement method (1)>. Among them, the divalent cation is preferably Mn²⁺. The solution including a divalent cation may be any solution in which a salt, a hydrate or the like containing the divalent cation is dissolved, and there are no particular limitations. Furthermore, the solution may also be a dilution that has been diluted with a buffer solution such as physiological saline, or with distilled water or the like. The enzymatic activity of arginase can be increased by adding a solution including a divalent cation to a sample including arginase.

The concentration of the divalent cation in the solution may be, for example, from 1 mM to 100 mM, may be from 3 mM to 50 mM, and may be from 5 mM to 30 mM.

The reaction vessel is not particularly limited as long as the arginase activity can be measured with the reaction vessel, and examples include reaction vessels similar to those listed as examples in the section <Arginase activity measurement method (1)>. The shape of the reaction vessel is not particularly limited, and examples include shapes similar to those listed as examples in the section <A rginase activity measurement method (1)>.

A mong them, a microplate shape is preferred because a large number of samples can be measured. An example of the microplate may be a microplate having an arbitrary number of wells disposed therein. The number of wells may be, for example, 24, 96, 384, or 1,536, per sheet of the plate. The reaction vessel may constitute a micro-flow channel device equipped with fine flow channels. The size of the reaction vessel does not matter as long as the size is in the range that is applicable to the apparatus used.

### [E nzyme-substrate reaction step]

Next, an arginine solution is added as a substrate to the sample including activated arginase, and an enzyme-substrate reaction is carried out. The reaction temperature is preferably from 20° C to 45° C, more preferably from 30° C to 40° C, and most preferably from 37° C to 40° C. The reaction time is preferably from 30 mi nutes to 3 hours, and particularly preferably 2 hours. The pH at the ti me of the reaction is preferably a pH of from 8.5 to 10.5, and particularly preferably a pH of from 9.0 to 10.0.

The arginine solution may be any solution including arginine (preferably, L-arginine), and there are no particular limitations. The arginine solution can be produced by dissolving a salt or a hydrate including arginine in a solvent such as distilled water. Furthermore, the arginine solution may also be a dilution that has been diluted with a buffer solution such as physiological saline, or with distilled water or the like.

### [Enzyme deactivation and urea detection reaction step]

Next, an acidic solution including a-isonitrosopropiophenone as a urea detection reagent is added to the sample subjected to the enzyme-substrate reaction, and the deactivation of enzyme and the urea detection reaction are simultaneously performed.

The acidic solution includes a-isonitrosopropiophenone as a urea detection reagent.

The pH of the acidic solution may be 4.0 or lower, and a solution having strong acidity, for which it is difficult to measure the pH using a pH meter having a glass electrode, may also be used. Examples of a usable acid include the acids similar to those listed as examples in the section <Arginase activity measurement method (1)>. A mixture of two or more kinds of the above-mentioned acids may also be used. Furthermore, the acidic solution is a dilution that has been diluted with a buffer solution such as physiological saline, or with distilled water or the like.

Since the acidic solution including a-isonitrosopropiophenone as a urea detection reagent is in a state of having been mixed in advance, the deactivation of enzyme and the detection of urea can be carried out by a single ti me of operation, and therefore, the operation can be made more efficient. Also, the amount of addition of the solution can be reduced, and the variation between the amount of addition of the solution and the amount of additi on of the urea detecti on reagent can be reduced. When the acidic solution including a-isonitrosopropiophenone as a urea detection reagent is combined with heating for accelerating the urea detection reaction, the enzyme reaction and the deactivation of enzyme can be sufficiently carried out.

The acidic solution including a-isonitrosopropiophenone as a urea detection reagent can be produced by, for example, the following method. First, a -isonitrosopropiophenone and an acid, or a salt or a hydrate of the acid, are mixed, together with a buffer solution such as physiological saline, or with distilled water or the like, as necessary. Furthermore, in a case in which it is relatively difficult for a-isonitrosopropiophenone to be dissolved in the acidic solution, it is preferableto heat the mixture at the ti me of mixing. By heati ng, a -isonitrosopropiophenone can be completely dissolved, and an acidic solution including a-isonitrosopropiophenone as a urea detection reagent can be produced.

T he concentration of the urea detection reagent in the solution is preferably from 0.05% by weight to 1.00% by weight, more preferably from 0.08% by weight to 0.70% by weight, and even more preferably from 0.10% by weight to 0.66% by weight.

### [Urea detecti on reaction accelerating step]

After the addition of the acidic solution including a-isonitrosopropiophenone as a urea detection reagent, the urea detection reaction may be accelerated by incubating the reaction system while heati ng the system. The heati ng temperature is preferably from room temperature to 100°C, and particularly preferably from 90°C to 100°C. The heating time is 30 minutes or longer, and is particularly preferably from 30 minutes to 12 hours. The heating temperature and the heating time can be regulated as appropriate according to the content of arginase. Furthermore, when the reaction system is incubated while being heated, the reaction caused by the urea detection reagent can be accelerated, and thus detection can be performed more rapidly.

Detection of urea can be carried out using a colorimetric method of utilizing a reaction between urea and the urea detection reagent. When the reaction proceeds and color is sufficiently developed, the reaction system is mounted in the measuring apparatus, and the absorbance is measured. The measuring apparatus is not particularly limited as long as the measuring apparatus can measure absorbance. Examples include an absorptiometer (spectrophotometer) and a microplate reader.

### [Measurement value calibration step]

Furthermore, the measurement value of the arginase activity may also be calibrated by measuring the protei n concentrati on in the sample.

The method for measuring the protein concentration is not particularly limited, and examples include measurement methods similar to those listed as examples in the section <Arginase activity measurement method (1)>.

When the protein concentration in the sample is measured, and the measurement value is cal i brated, a more accurate measurement value of arginase activity can be obtained.

T he screeni ng method of the present embodi ment is capable of maki ng measurement efficiently by using small amounts of samples and reagents, as disclosed above. There has been hitherto a problem that the urea detection reagent is discolored over ti me and the background value becomes high; however, in the screeni ng method of the present embodiment, an acidic solution including a urea detection reagent and having a pH in the range described above can be produced in advance and stored, and thus, measurement can be made with higher sensitivity in a state in which the background value is low. T herefore, while measurement is limited to several dozens of samples in a single time of analysis in the conventional methods, a large number of samples such as several hundred samples or more can be measured in the screeni ng method of the present embodi ment.

### [Determination step]

Next, in a case in which the concentration of urea detected in the presence of the test substance is low compared to the concentration of urea detected in the absence of the test substance, it can be determined that the test substance is an i nhi bitor of arginase.

Meanwhile, in a case in which the concentration of urea detected in the presence of the test substance is high compared to the concentration of urea detected in the absence of the test substance, it can be determined that the test substance is an active agent of arginase.

### EXAMPLES

Hereinafter, the present invention will be explained by way of Examples; however, the invention is not intended to be limited to the following Examples.

### [Example 1]

### (1) A rgi nase activati on step

20 mL each of a mixed liquid of a 50 mM Tris-HCl (pH 7.5) and a 10 mM MnCl₂ was dispensed onto a microplate using a multipipettor. Subsequently, animal cells were disrupted by using a mixed liquid of a 0.1% Triton X 100 and a Proteinase Inhibitor Mixture, and thus a disrupted cell suspension was produced as a sample including arginase.

Subsequently, the disrupted cell suspension thus produced was added to the microplate in an amount of 20 mL each by using a multi pi pettor. Subsequently, the microplate was incubated for 10 mi nutes at 56° C, and thereby arginase was activated.

### (2) E nzyme-substrate reaction step

Subsequently, a 0.5 M arginine solution (pH 9.7) as a substrate was added to the microplate in an amount of 40 mL each by using a multipipettor. The microplate was incubated for 2 hours at 37° C, and thus an enzyme-substrate reaction was carried out.

### (3) Enzyme deactivation and urea detection reaction step

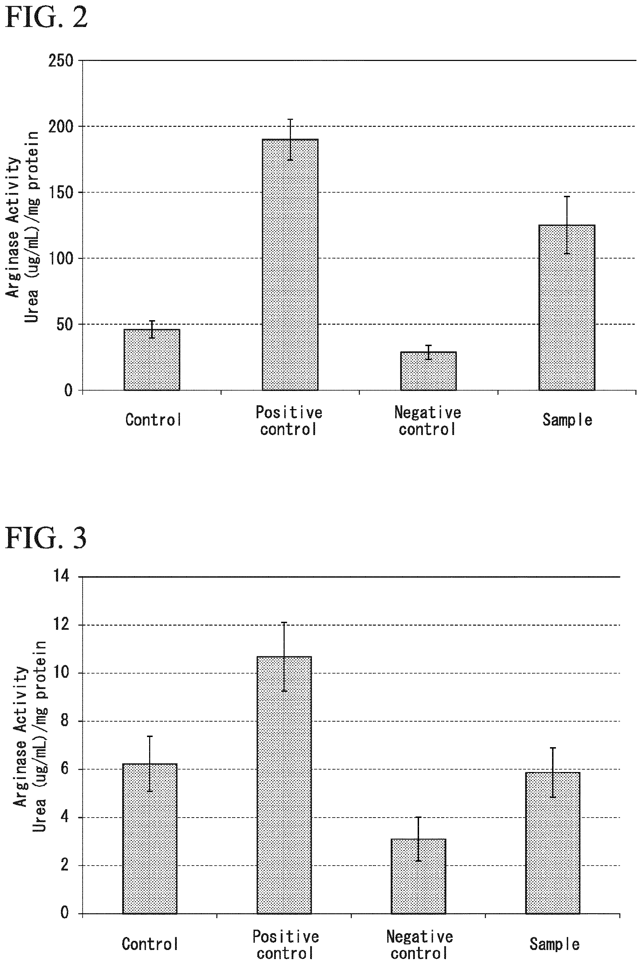

To an acid mixed liquid (phosphoric acid:concentrated sulfuric acid:water = 1:3:7 (volume ratio)), a-isonitrosopropiophenone(9% by mass, dissolved in 100% ethanol) was added in an amount equivalent to 1/10 of the amount of the acid mixed liquid, and the mixture was heated and dissolved at 95° C for 30 minutes or longer. Thus, an acidic solution including the urea detection reagent was produced. Subsequently, the acidic solution including the urea detection reagent thus produced was added to the microplate in an amount of 180 mL each by using a multi pi pettor. Subsequently, the microplate was incubated overnight at normal temperature. Subsequently, the microplate was heated at 95° C for 1 hour and 45 mi nutes. Subsequently, the absorbance at 540 nm of the sample including arginase was measured using a microplate reader (manufactured by Molecular Devices, LLC., SPECTRA MAX i3), for which a calibration curve had been produced in advance with urea solutions at 0, 5, 10, 20, 40, 80, and 160 rrg/mL. Furthermore, the measurement value was cal i brated by using the protein concentration of the sample including arginase, which had been measured in advance by BCA protein assay. The results are presented in FIG. 2.

In FIG. 2, Control represents a sample subjected to the enzyme-substrate reaction and the urea detection reaction using a disrupted cell suspension that did not contain anything; Positive Control represents a sample subjected to the enzyme-substrate reaction and the urea detection reaction using a disrupted cell suspension, to which an arginase activity promoting factor had been added; Negative Control represents a sample subjected to the enzyme-substrate reaction and the urea detection reaction using a disrupted cell suspension, to which an arginase activity inhibiting factor had been added; and Sample represents a sample subjected to the enzyme-substrate reaction and the urea detection reaction using a disrupted cell suspension, to which an arginase activity promoti ng factor and an arginase activity inhibiting factor had been added.

### [Comparative Example 1]

### (1) E nzyme-substrate reaction step

A five-fold concentrated substrate buffer was produced by mixing an arginine buffer (manufactured by BioAssay Systems, LLC., QUANTICHROM (registered trademark) A rginaseAssay Kit(DARG-200)) and a M n solution (manufactured by BioAssay Systems, LLC., QUANTICHROM (registered trademark) ArginaseAssay Kit (DARG-200)) at a ratio of 4:1. Subsequently, a disrupted cell suspension produced by a method similar to that of Example 1 was dispensed onto a microplate in an amount of 40 mL each. Subsequently, a five-fold concentrated substrate buffer was dispensed onto the microplate in an amount of 10 mL each. Furthermore, as a blank, wells containing only the disrupted cell suspension without dispensing the five-fold concentrated substrate buffer, were also prepared. The microplate was incubated for 2 hours at 37·C, and thus an enzyme-substrate reaction was carried out.

### (2) Enzyme deactivation and urea detection reaction step

A urea detection reagent was produced by mixing reagent A (manufactured by BioAssay Systems, LLC., QUANTICHROM (registered trademark) ArginaseAssay Kit (DARG-200)) and reagent B (manufactured by BioAssay Systems, LLC., QUANTICHROM (registered trademark) A rginaseAssay Kit (DARG-200)) at a ratio of 1:1. Subsequently, the urea detection reagent was added to a microplate in an amount of 200 mL each so as to stop the enzyme reaction, and a urea detection reaction was initiated. At this ti me, 10 mL of a five-fold concentrated substrate buffer was added to the blank. Subsequently, the microplate was incubated for 60 mi nutes at room temperature. Subsequently, the absorbance at 430 nm of the sample including arginase was measured using a microplate reader (manufactured by Molecular Devices, LLC., SPECTRA MAX i3), for which a calibration curve had been produced in advance with urea solutions at 0, 5, 10, 20, 40, 80, and 160 rrg/mL. The results are presented in FIG. 3.

In FIG. 3, Control represents a sample subjected to the enzyme-substrate reaction and the urea detection reaction using a disrupted cell suspension that did not contain anythi ng; Positive Control represents a sample subjected to the enzyme-substrate reaction and the urea detection reaction using a disrupted cell suspension, to which an arginase activity promoting factor had been added; Negative Control represents a sample subjected to the enzyme-substrate reaction and the urea detection reaction using a disrupted cell suspension, to which an arginase activity inhibiting factor had been added; and Sample represents a sample subjected to the enzyme-substrate reaction and the urea detection reaction using a disrupted cell suspension, to which an arginase activity promoting factor and an arginase activity inhibiting factor had been added.

From FIG. 2 and FIG. 3, with respect to the measurement value of arginine activity of the Negative Control, the measurement value of arginine activity of the Sample was two times in Comparative Example 1, while the measurement value of arginine activity of the Sample was five times in Example 1. Furthermore, in Comparative Example 1, a significant difference between the measurement value of arginine activity in the Control and the measurement value of arginine activity in the Sample was not obtained; however, in Example 1, significant differences were obtained between any conditions. It is speculated that in the arginase activity measurement method described in Comparative Example 1, the urea detection reagent was discolored over time, and the background value became high.

F rom the results given above, it was confirmed that in the arginase activity measurement method described in Example 1, the background value is low, and the arginase activity can be measured with high sensitivity.

### [Example 2]

### (1) Production of urea solutions

Urea solutions were produced using urea (manufactured by Nacalai Tesque, Inc.) so as to obtain concentrati ons of 0, 5, 10, 20, 40, 80, and 160 mg/mL.

### (2) Production of acidic solution including urea detection reagent

To an acid mixed liquid (phosphoric acid:concentrated sulfuric acid: water = 1:3:7 (volume ratio)), a-isonitrosopropiophenone(9% by mass, dissolved in 100% ethanol) was added in an amount equivalent to 1/10 of the amount of the acid mixed liquid, and the mixture was heated and dissolved at 95·C for 30 minutes or longer. Thus, an acidic solution including the urea detection reagent was produced.

### (3) Urea detection reaction step

To one sheet of a microplate, 20 mL each of the urea solutions at different concentrations produced in (1), 20 mL each of a manganese solution, and 40 mL each of an arginine solution were respectively added, each solution being added to eight wells (56 wells in total ¼ 1 sheet), using a multipipettor. Subsequently, the acidic solution including the urea detection reagent produced in (2) was added to the microplate in an amount of 180 mL each, using a multi pi pettor. Subsequently, the microplate was heated at 95·C for 2, 3, 4, 5, 6, or 8 hours. Subsequently, the absorbance at 540 nm was measured using a microplate reader (manufactured by Molecular Devices, LLC., SPECTRA MAX i3). The results are presented in Table 1.

In Table 1, "mean" represents a value obtained by first calculating the average value of the eight wells for each of the urea concentrations of 0, 5, 10, 20, 40, 80, and 160 mg/mL, subsequently calculating the value of the average value divided by the standard deviation (average value / standard deviation), and averaging the values of the other seven calculated values of average value / standard deviation. Furthermore, "SD" represents the standard deviation of the seven values of the average value/standard deviation.

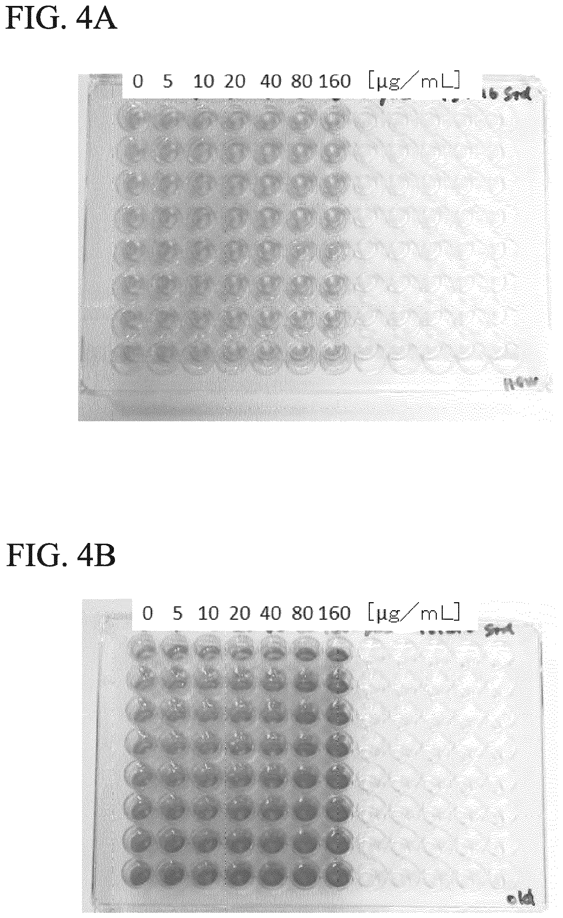

A captured image showing the liquid color of the solutions in the microplate that was heated for 8 hours is presented in FIG. 4A.

### [Comparative Example 2]

### (1) Production of urea solutions

Urea solutions were produced using urea (manufactured by Nacalai Tesque, Inc.) by a method similar to that of secti on (1) of Example 2, so as to obtain concentrati ons of 0, 5, 10, 20, 40, 80, and 160 rrg/mL.

### (2) Urea detection reaction step

To one sheet of a microplate, 20 mL each of the urea solutions at different concentrations produced in (1), 20 mL each of a manganese solution, and 40 mL each of an arginine solution were respectively added, each solution being added to eight wells (56 wells in total ¼ 1 sheet), using a multipipettor. Subsequently, an acid mixed liquid (phosphoric acid:concentrated sulfuric acid:water = 1:3:7 (volume ratio)) was added to the microplate in an amount of 162 mL each, using a multipipettor. Subsequently, a-isonitrosopropiophenone(9% by mass, dissolved in 100% ethanol) as a urea detection reagent was added in an amount of 18 mL each, using a multi pi pettor. The microplate was heated at 95·C for 2, 3, 4, 5, 6, or 8 hours. Subsequently, the absorbance at 540 nm was measured using a microplate reader (manufactured by Molecular Devices, LLC., SPECTRA MAX i3). The results are presented in Table 1.

A captured image showi ng the liquid color of the solutions in the microplate that was heated for 8 hours is presented in FIG. 4B.

**[Table 1]**

| Heating time [h] | Example 2 | | Comparative Example 2 | |
|---|---|---|---|---|
| | mean | SD | mean | SD |
| 2 | 0.02574 | 0.009296 | 0.37743 | 0.199294 |
| 3 | 0.02406 | 0.009609 | 0.30748 | 0.174363 |
| 4 | 0.02498 | 0.008290 | 0.24349 | 0.140597 |
| 5 | 0.02928 | 0.011491 | 0.05009 | 0.023409 |
| 6 | 0.03484 | 0.011253 | 0.04777 | 0.019443 |
| 8 | 0.03604 | 0.025473 | 0.04891 | 0.016640 |

From Table 1, Example 2 tended to have smallervalues of the mean, compared to Comparative Example 2. Meanwhile, in Comparative Example 2, the values of the mean were dispersed over different heating times. Furthermore, in Example 2, the values of SD also tended to be smaller compared to Comparative Example 2, except for the value obtained after heati ng for 8 hours.

From the above results, it became clear that in the measurement method of the present invention, the variation between measurement values can be reduced.

From FIG. 4A and FIG. 4B, while discoloration of the urea detection reagent was hardly observed after 8 hours in Example 2, discoloration of the urea detection reagent after 8 hours was recognized in Comparative Example 2. This is speculated to be because, in Comparative Example 2, the ethanol solvent included in the urea detection reagent is evaporated during the dispensing operation, and the concentration of a-isonitrosopropiophenone becomes higher; whereas in Example 2, since an acidic solution including the urea detection reagent had been mixed in advance and then was dispensed, the subsequent process could be carried out without having the ethanol solvent evaporated.

Therefore, it became clear that in Comparative Example 2, which is a conventional measurement method, the background value is high; however, in the measurement method of the present invention, the background value is low, and the arginase activity can be detected more accurately.

### [Example 3]

### (1) A rgi nase activati on step

A 10 mM MnCl₂ solution was dispensed on a microplate in an amount of 20 mL each, using a multi pi pettor. Subsequently, mouse macrophage RAW264 cells were disrupted using a mixed liquid of 0.1% Triton X 100 and Proteinase Inhibitor Mixture, and thus, a disrupted cell suspension was produced as a sample including arginase. Subsequently, the disrupted cell suspension thus produced was added to the microplate in an amount of 20 mL each, using a multipipettor.

### (2) E nzyme-substrate reaction step

Subsequently, a 0.5 M arginine solution (pH 9.7) was added as a substrate to the microplate in an amount of 40 mL each, using a micropi pettor. The microplate was incubated at 37·C for 2 hours, and thus an enzyme-substrate reaction was carried out.

### (3) Enzyme deactivation and urea detection reaction step

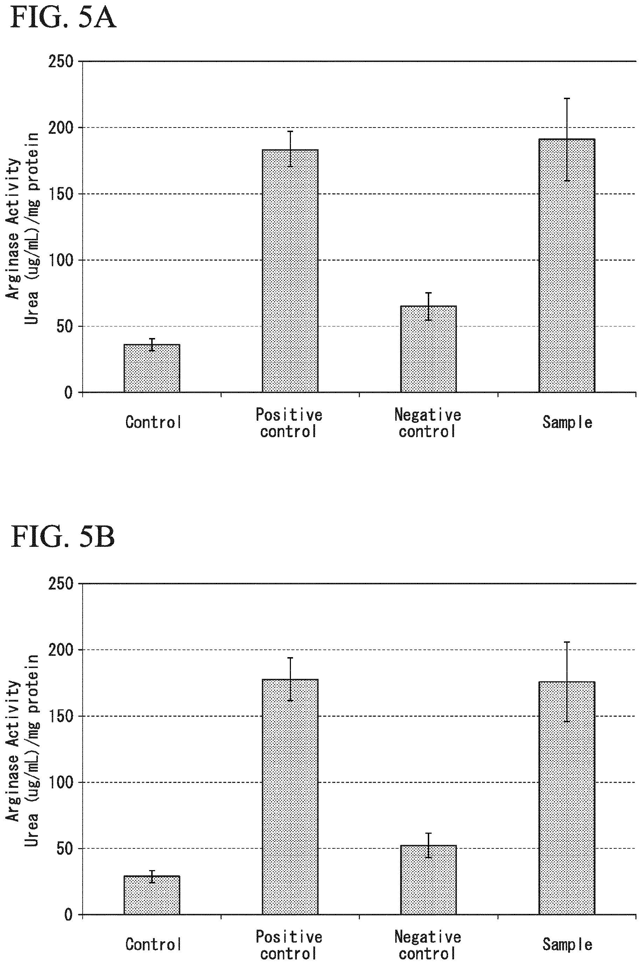

To an acid mixed liquid (phosphoric acid:concentrated sulfuric acid:water = 1:3:7 (volume ratio)), a-isonitrosopropiophenone(9% by mass, dissolved in 100% ethanol) was added in an amount equivalent to 1/10 of the amount of the acid mixed liquid, and the mixture was heated and dissolved at 95·C for 30 minutes or longer. Thus, an acidic solution including the urea detection reagent was produced. Subsequently, the acidic solution including the urea detection reagent thus produced was added to the microplate in an amount of 180 mL each by using a multi pi pettor. Subsequently, the microplate was incubated overnight at normal temperature. Subsequently, the microplate was heated at 95·C for 1 hour and 45 mi nutes or for 2 hours and 15 minutes. Subsequently, the absorbance at 540 nm of the sample including arginase was measured using a microplate reader (manufactured by Molecular Devices, LLC., SPECTRA MAX i3), for which a calibration curve had been produced in advance with urea solutions at 0, 5, 10, 20, 40, 80, and 160 mg/mL. Furthermore, the measurement value was calibrated by using the protein concentration of the sample including arginase, which had been measured in advance by BCA protein assay. The results are presented in FIG. 5A and FIG. 5B.

In FIG. 5A and FIG. 5B, Control represents a sample subjected to the enzyme-substrate reaction and the urea detection reaction using a disrupted cell suspension that did not contain anything; Positive Control represents a sample subjected to the enzyme-substrate reaction and the urea detection reaction using a disrupted cell suspension, to which IL-4 as an arginase activity promoting factor had been added; Negative Control represents a sample subjected to the enzyme-substrate reaction and the urea detection reaction using a disrupted cell suspension, to which Lipopolysaccharide (LPS) and INTERFERON g (IFNg) as arginase activity inhibiting factors had been added; and Sample represents a sample subjected to the enzyme-substrate reaction and the urea detection reaction using a disrupted cell suspension, to which an arginase activity promoti ng factor (IL-4) and arginase activity inhibiting factors (L PS and IF Ng) had been added.

### [Comparative Example 3]

### (1) E nzyme-substrate reaction step

A disrupted cell suspension produced using a method similar to that of Example 3 was dispensed to the microplate in an amount of 20 mL each, and an enzyme-substrate (L-arginine 40 mL + manganese chloride 20 mL) solution was dispensed in an amount of 60 mL each. Furthermore, as blanks, wells containing the enzyme-substrate solution only and wells containing manganese chloride only were also prepared. The microplate was incubated at 37·C for 60 minutes, and an enzyme-substrate reaction was carried out.

### (2) Enzyme deactivation and urea detection reaction step

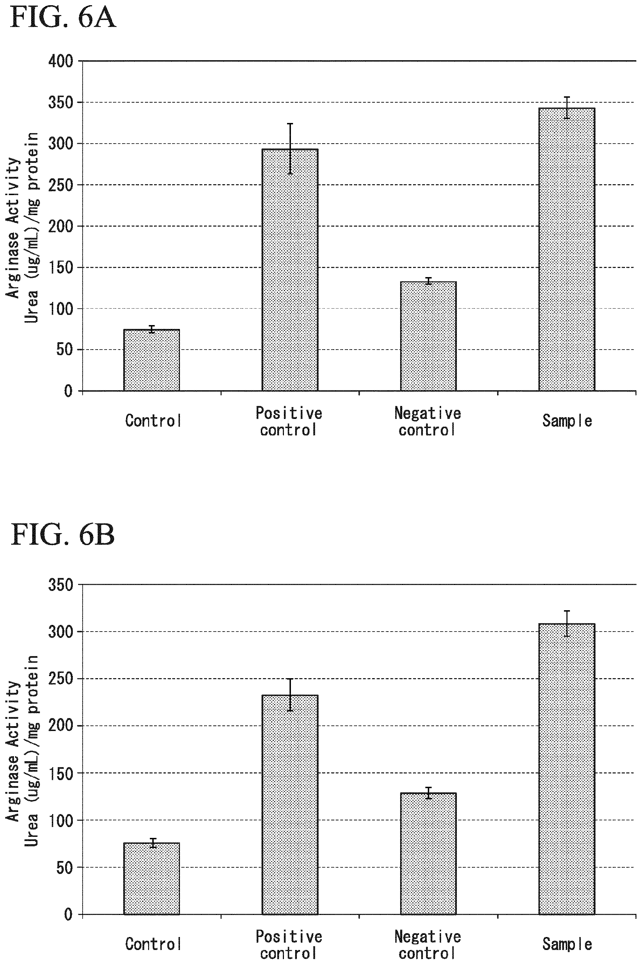

Subsequently, a urea detection reagent was produced by mixing reagent A (10 mM o-phthaldialdehyde and 0.4% polyoxyethylene(23) lauryl ether (w/v) in 1.8 M sulfuric acid) and reagent B (1.3 mM primaquine diphosphate, 0.4% polyoxyethylene(23) lauryl ether (w/v), and 130 mM boric acid in 3.6 M sulfuric acid) at a ratio of 1:1. Subsequently, the urea detection reagent was added to the microplate in an amount of 180 mL each immediately after mixing of reagent A and reagent B to stop the enzyme reaction, and a urea detection reaction was initiated. Subsequently, the microplate was left to stand for 10 mi nutes or 120 mi nutes at room temperature, and thus color was developed. Subsequently, the absorbance at 430 nm of the sample including arginase was measured using a microplate reader (manufactured by Molecular Devices, LLC., SPECTRA MAX i3), for which a calibration curve had been produced in advance with urea solutions at 0, 5, 10, 20, 40, 80, and 160 rrg/mL. The results are presented in FIG. 6A and FIG. 6B.

In FIG. 6A and FIG. 6B, Control represents a sample subjected to the enzyme-substrate reaction and the urea detection reaction using a disrupted cell suspension that did not contain anything; Positive Control represents a sample subjected to the enzyme-substrate reaction and the urea detection reaction using a disrupted cell suspension, to which IL-4 as an arginase activity promoting factor had been added; Negative Control represents a sample subjected to the enzyme-substrate reaction and the urea detection reaction using a disrupted cell suspension, to which Lipopolysaccharide (LPS) and INTERFERON g (IFNg) as arginase activity inhibiting factors had been added; and Sample represents a sample subjected to the enzyme-substrate reaction and the urea detection reaction using a disrupted cell suspension, to which an arginase activity promoti ng factor (IL-4) and arginase activity inhibiting factors (L PS and IF Ng) had been added.

From FIG. 5A and FIG. 5B, there was no significant difference between the measurement values of arginine activity of the various samples in the case where the heati ng ti me was 1 hour and 45 mi nutes and in the case where the heati ng ti me was 2 hours and 15 mi nutes.

Furthermore, from FIG. 6A and FIG. 6B, there was no significant difference between the measurement value of arginine activity of the various samples in the case where the color development ti me was 10 mi nutes and in the case where the color devel opment time was 120 mi nutes.

Meanwhile, with respect to the measurement value of arginine activity of the Negative Control, the measurement value of arginine activity of the Sample was about 2.4 to 2.6 times in Comparative Example 3, while the measurement value of arginine activity of the Sample was higher, such as about 2.9 to 3.3 times, in Example 3.

Furthermore, while the measurement value of argi ni ne activity in the Control was about 75 in Comparative Example 3, the measurement value of arginine activity in the Control was about 29 to 37, which was 50 or less, in Example 3.

From these results, it is speculated that in the arginase activity measurement method described in Comparative Example 3, the urea detection reagent was discolored as the reaction proceeded immediately after the mixing of reagent A and reagent B, and the background value became high.

F rom the results given above, it was confirmed that in the arginase activity measurement method described in Example 3, a large number of samples can be measured by a si ngle ti me of measurement, and thus, even in a case in which a waiti ng ti me for samples is needed until measurement is made, the background value is low, and the arginase activity can be measured with high sensitivity.

### [Example 4]

### (1) Arginase activation step

A 10 mM MnCl₂ solution was dispensed onto a microplate in an amount of 20 mL each, using a multi pi pettor. Subsequently, mouse macrophage RAW264 cells were disrupted using a mixed liquid of 0.1% Triton X 100 and Proteinase Inhibitor Mixture, and thus a disrupted cell suspension was produced as a sample including arginase. Subsequently, the disrupted cell suspension thus produced was added to the microplate in an amount of 20 mL each, using a multi pi pettor.

### (2) E nzyme-substrate reaction step

Subsequently, a 0.5 M arginine solution (pH 9.7) was added as a substrate to the microplate in an amount of 40 mL each, using a multipipettor. The microplate was incubated at 37·C for 2 hours, and thus an enzyme-substrate reaction was carried out.

### (3) Enzyme deactivation and urea detection reaction step

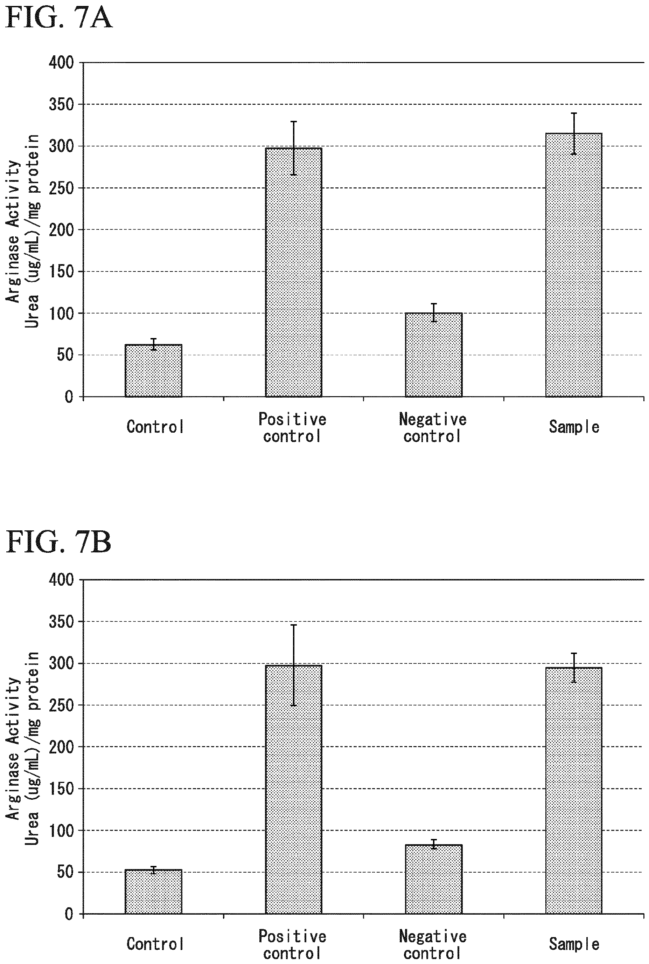

To an acid mixed liquid (phosphoric acid:concentrated sulfuric acid:water = 1:3:7 (volume ratio)), a-isonitrosopropiophenone(9% by mass, dissolved in 100% ethanol) was added in an amount equivalent to 1/10 of the amount of the acid mixed liquid, and the mixture was heated and dissolved at 95·C for 30 minutes or longer. Thus, an acidic solution including the urea detection reagent was produced, and the acidic solution was stored for 24 hours at room temperature. Subsequently, the acidic solution including the urea detection reagent, which had been stored for 24 hours at room temperature after production, was added to the microplate in an amount of 180 mL each by using a multi pi pettor. Subsequently, the mi croplate was incubated overnight at normal temperature. Subsequently, the microplate was heated at 95·C for 1 hour and 45 mi nutes or 2 hours and 15 mi nutes. Subsequently, the absorbance at 540 nm of the sample including arginase was measured using a microplate reader (manufactured by Molecular Devices, LLC., SPECTRA MAX i3), for which a calibration curve had been produced in advance with urea solutions at 0, 5, 10, 20, 40, 80, and 160 mg/mL. Furthermore, the measurement value was cal i brated by using the protei n concentrati on of the sample including arginase, which had been measured in advance by BCA protein assay. The results are presented in FIG. 7A and FIG. 7B.

In FIG. 7A and FIG. 7B, Control represents a sample subjected to the enzyme-substrate reaction and the urea detection reaction using a disrupted cell suspension that did not contain anything; Positive Control represents a sample subjected to the enzyme-substrate reaction and the urea detection reaction using a disrupted cell suspension, to which IL-4 as an arginase activity promoting factor had been added; Negative Control represents a sample subjected to the enzyme-substrate reaction and the urea detection reaction using a disrupted cell suspension, to which Lipopolysaccharide (LPS) and INTERFERON g(IFNg) as arginase activity inhibiting factors had been added; and Sample represents a sample subjected to the enzyme-substrate reaction and the urea detection reaction using a disrupted cell suspension, to which an arginase activity promoti ng factor (IL-4) and arginase activity inhibiting factors (L PS and IF Ng) had been added.

### [Comparative Example 4]

### (1) E nzyme-substrate reaction step

A disrupted cell suspension produced by using a method similar to that of Example 3 was dispensed onto a microplate in an amount of 20 mL each, and an enzyme-substrate (L-argi ni ne 40 mL + manganese chloride 20 mL) solution was dispensed in an amount of 60 mL each. Furthermore, as blanks, wells containing the enzyme-substrate solution only and wells containing manganese chloride only were also prepared. The microplate was incubated at 37·C for 60 mi nutes, and an enzyme-substrate reaction was carried out.

### (2) E nzyme deactivation and urea detection reaction step

Subsequently, a urea detection reagent was produced by mixing reagentA (10 mM o-phthaldialdehyde and 0.4% polyoxyethylene(23) lauryl ether (w/v) in 1.8 M sulfuric acid) and reagent B (1.3 mM primaquine diphosphate, 0.4% polyoxyethylene(23) lauryl ether (w/v), and 130 mM boric acid in 3.6 M sulfuric acid) at a ratio of 1:1. The urea detection reagent was stored for 24 hours at 4·C. Subsequently, the urea detection reagent that had been stored for 24 hours at 4·C after production was added to the microplate in an amount of 180 mL each so as to stop the enzyme reaction, and a urea detecti on reaction was i niti ated. S ubsequently, the mi croplate was left to stand for 10 mi nutes or 120 mi nutes at room temperature, and thus color was developed. Subsequently, the absorbance at 430 nm was measured using a microplate reader (manufactured by Molecular Devices, LLC., SPECTRA MAX i3); however, a calibration curve could not be produced with the urea solutions at 0, 5, 10, 20, 40, 80, and 160 rrg/mL .

The absorbance was measured for four kinds of samples including argi nase, that is, a sample subjected to the enzyme-substrate reaction and the urea detection reaction using a disrupted cell suspension that did not contain anything (Control); a sample subjected to the enzyme-substrate reaction and the urea detection reaction using a disrupted cell suspension, to which IL-4 as an arginase activity promoting factor had been added (Positive Control); a sample subjected to the enzyme-substrate reaction and the urea detection reaction using a disrupted cell suspension, to which Lipopolysaccharide(LPS) and INTERFERON g(IFNg) as arginase activity inhibiting factors had been added (Negative Control); and a sample subjected to the enzyme-substrate reaction and the urea detection reaction using a disrupted cell suspension, to which an arginase activity promoting factor (IL-4) and argi nase activity inhibiting factors (L PS and IFNg) had been added (Sample). However, the absorbance results did not reflect the various conditions and exhibited a tendency that was completely different from the results of Comparative Example 3.

From FIG. 7A and FIG. 7B, there was no significant difference between the measurement values of arginine activity of the various samples in the case where the heati ng ti me was 1 hour and 45 mi nutes and in the case where the heati ng ti me was 2 hours and 15 mi nutes.

With respect to the measurement value of argi ni ne activity of the Negative Control, the measurement value of arginine activity of the Sample did not reflect the various conditions in Comparative Example 4, and a comparison could not be made. However, the measurement value of arginine activity of the Sample was higher, such as about 3.1 to 3.5 times, in Example 4.

From these results, it is speculated that in the arginase activity measurement method described in Comparative Example 4, the reaction proceeded due to the lapse of 24 hours after mixing of reagent A and reagent B, and the arginase activity was saturated, whereby the acidic solution had completely lost the function as a urea detection reagent.

On the other hand, although specific data are not shown, in the arginase activity measurement method described in Example 4, the acidic solution including the urea detection reagent thus produced can be used for the measurement of arginase activity without any problem, even if the storage period is longer than 24 hours (for example, about several months).

From the results given above, it was confirmed that the acidic solution including a urea detection reagent that was used for the arginase activity measurement method described in Example 4 can be produced in large quantities, can be stored for a long time period, exhibits a low background value even after a long-term storage, and can measure the arginase activity with high sensitivity.

### Industrial Applicability

According to the present invention, the arginase activity can be measured simply and conveniently with high sensitivity.

### Reference Signs List

1: sample including arginase, 2: solution including divalent cation, 3: reaction vessel, 4: argi nine solution, 5: acidic solution including urea detection reagent and having pH of from 1.0 to 4.0, 6: measuring apparatus

## Claims

1. An arginase activity measurement method, comprising:
a step of adding a sample including arginase and a solution including a divalent cation to a reaction vessel, and thereby activating arginase;
a step of adding an arginine solution to the sample including the activated arginase, and performing an enzyme-substrate reaction; and
a step of adding an acidic solution including α-isonitrosopropiophenone as a urea detection reagent and physiological saline or distilled water to the sample subjected to the enzyme-substrate reaction, and simultaneously performing deactivation of enzyme and a urea detection reaction.

2. The arginase activity measurement method according to Claim 1, further comprising a step of heating the sample to which the acidic solution is added at a temperature higher than or equal to a room temperature and lower than or equal to 100°C for 30 minutes or longer, and accelerating the urea detection reaction.

3. The arginase activity measurement method according to Claim 1 or 2, further comprising a step of measuring a protein concentration in the sample, and calibrating a measurement value of arginase activity.

4. The arginase activity measurement method according to any one of Claims 1 to 3, wherein the reaction vessel is a microplate.

5. The arginase activity measurement method according to any one of Claims 1 to 4, wherein a large number of samples can be measured in a single time of analysis.

6. An arginase activity detection kit, comprising an acidic solution including α-isonitrosopropiophenone as a urea detection reagent and physiological saline or distilled water.

7. An arginase inhibitor or active agent screening method, comprising:
a step of adding a sample including arginase and a solution including a divalent cation to a reaction vessel in the presence or absence of a test substance, and thereby activating arginase;
a step of adding an arginine solution to the sample including the activated arginase, and performing an enzyme-substrate reaction;
a step of adding an acidic solution including α-isonitrosopropiophenone as a urea detection reagent and physiological saline or distilled water to the sample subjected to the enzyme-substrate reaction, and simultaneously performing deactivation of enzyme and a urea detection reaction; and
a step of determining that in a case in which the concentration of urea detected in the presence of the test substance is low compared to the concentration of urea detected in the absence of the test substance, the test substance is an inhibitor of arginase, and
determining that in a case in which the concentration of urea detected in the presence of the test substance is high compared to the concentration of urea detected in the absence of the test substance, the test substance is an active agent of arginase.

## Patentansprüche

1. Arginaseaktivitätsmessverfahren, umfassend:
einen Schritt eines Hinzufügens einer Probe, die Arginase umfasst, und einer Lösung, die ein zweiwertiges Kation umfasst, zu einem Reaktionsgefäß und dadurch Aktivieren von Arginase;
einen Schritt eines Hinzufügens einer Argininlösung zu der Probe, die die aktivierte Arginase umfasst, und eines Durchführens einer Enzym-Substrat-Reaktion; und
ein Schritt eines Hinzufügens einer sauren Lösung, die α-Isonitrosopropiophenon als ein Harnstoffnachweisreagenz und physiologische Kochsalzlösung oder destilliertes Wasser umfasst, zu der Probe, die der Enzym-Substrat-Reaktion unterworfen wird, und eines gleichzeitigen Durchführens einer Deaktivierung von Enzym und einer Harnstoffnachweisreaktion.

2. Arginaseaktivitätsmessverfahren nach Anspruch 1, ferner umfassend einen Schritt eines Erhitzens der Probe, zu der die saure Lösung gegeben ist, bei einer Temperatur, die höher oder gleich einer Raumtemperatur und niedriger als oder gleich 100 °C ist für 30 Minuten oder länger und eines Beschleunigens der Harnstoffnachweisreaktion.

3. Arginaseaktivitätsmessverfahren nach Anspruch 1 oder 2, ferner umfassend einen Schritt eines Messens einer Proteinkonzentration in der Probe und eines Kalibrierens eines Messwerts der Arginaseaktivität.

4. Arginaseaktivitätsmessverfahren nach einem der Ansprüche 1 bis 3, wobei das Reaktionsgefäß eine Mikroplatte ist.

5. Arginaseaktivitätsmessverfahren nach einem der Ansprüche 1 bis 4, wobei eine große Anzahl von Proben in einer einzigen Analysezeit gemessen werden kann.

6. Arginaseaktivitätsnachweiskit, umfassend eine saure Lösung umfassend α-Isonitrosopropiophenon als ein Harnstoffnachweisreagenz und physiologische Kochsalzlösung oder destilliertes Wasser.

7. Ein Arginase-Inhibitor- oder -Wirkstoff-Screening-Verfahren, umfassend:
einen Schritt eines Hinzufügens einer Probe, die Arginase umfasst, und einer Lösung, die ein zweiwertiges Kation umfasst, zu einem Reaktionsgefäß in der Gegenwart oder Abwesenheit einer Testsubstanz und dadurch Aktivieren von Arginase;
einen Schritt eines Hinzufügens einer Argininlösung zu der Probe, die die aktivierte Arginase umfasst, und eines Durchführens einer Enzym-Substrat-Reaktion;
einen Schritt eines Hinzufügens einer sauren Lösung, die α-Isonitrosopropiophenon als ein Harnstoffnachweisreagenz und physiologische Kochsalzlösung oder destilliertes Wasser umfasst, zu der Probe, die der Enzym-Substrat-Reaktion unterworfen wird, und eines gleichzeitigen Durchführens einer Deaktivierung von Enzym und einer Harnstoffnachweisreaktion; und
einen Schritt eines Bestimmens, dass in einem Fall, in dem die in der Gegenwart der Testsubstanz nachgewiesene Harnstoffkonzentration im Vergleich zu der in der Abwesenheit der Testsubstanz nachgewiesenen Harnstoffkonzentration niedrig ist, die Testsubstanz ein Inhibitor von Arginase ist, und
eines Bestimmens, dass in einem Fall, in dem die in der Gegenwart der Testsubstanz nachgewiesene Harnstoffkonzentration im Vergleich zu der in der Abwesenheit der Testsubstanz nachgewiesenen Harnstoffkonzentration hoch ist, die Testsubstanz ein Wirkstoff von Arginase ist.

## Revendications

1. Procédé de mesure de l'activité de l'arginase, comprenant :
une étape consistant à ajouter un échantillon incluant de l'arginase et une solution incluant un cation divalent dans un récipient de réaction, et ainsi à activer l'arginase ;
une étape consistant à ajouter une solution d'arginine à l'échantillon incluant l'arginase activée, et à réaliser une réaction enzyme-substrat ; et
une étape consistant à ajouter une solution acide incluant une α-isonitrosopropiophénone en tant que réactif de détection de l'urée et du sérum physiologique ou de l'eau distillée à l'échantillon soumis à la réaction enzyme-substrat, et simultanément à réaliser la désactivation de l'enzyme et une réaction de détection de l'urée.

2. Procédé de mesure de l'activité de l'arginase selon la revendication 1, comprenant en outre une étape consistant à chauffer l'échantillon auquel la solution acide est ajoutée à une température supérieure ou égale à la température ambiante et inférieure ou égale à 100 °C pendant 30 minutes ou plus, et à accélérer la réaction de détection de l'urée.

3. Procédé de mesure de l'activité de l'arginase selon la revendication 1 ou 2, comprenant en outre une étape consistant à mesurer une concentration de protéine dans l'échantillon, et à étalonner une valeur de mesure de l'activité de l'arginase

4. Procédé de mesure de l'activité de l'arginase selon l'une quelconque des revendications 1 à 3, dans lequel le récipient de réaction est une microplaque.

5. Procédé de mesure de l'activité de l'arginase selon l'une quelconque des revendications 1 à 4, dans lequel un grand nombre d'échantillons peuvent être mesurés en une seule analyse.

6. Kit de détection de l'activité de l'arginase, comprenant une solution acide incluant une α-isonitrosopropiophénone en tant que réactif de détection de l'urée et du sérum physiologique ou de l'eau distillée.

7. Procédé de criblage d'agent actif ou d'inhibiteur de l'arginase, comprenant :
une étape consistant à ajouter un échantillon incluant de l'arginase et une solution incluant un cation divalent dans un récipient de réaction en présence ou en l'absence d'une substance test, et ainsi à activer l'arginase ;
une étape consistant à ajouter une solution d'arginine à l'échantillon incluant l'arginase activée, et à réaliser une réaction enzyme-substrat ;
une étape consistant à ajouter une solution acide incluant une α-isonitrosopropiophénone en tant que réactif de détection de l'urée et du sérum physiologique ou de l'eau distillée à l'échantillon soumis à la réaction enzyme-substrat, et simultanément à réaliser la désactivation de l'enzyme et une réaction de détection de l'urée ; et
une étape consistant à déterminer que dans le cas où la concentration d'urée détectée en présence de la substance test est faible comparée à la concentration d'urée détectée en l'absence de la substance test, la substance test est un inhibiteur de l'arginase ; et
à déterminer que dans le cas où la concentration d'urée détectée en présence de la substance test est élevée comparée à la concentration d'urée détectée en l'absence de la substance test, la substance test est un agent actif de l'arginase.
